# NEW EUROPEAN PATENT SPECIFICATION

(11) **EP 0 271 007 B2**
(45) Date of publication and mention of the opposition decision: **01.03.2000**
(45) Mention of the grant of the patent: 16.03.1994
(21) Application number: 87117915.6
(22) Date of filing: 03.12.1987
(51) Int. Cl.: C08G 61/08, C07C 13/61

(54) **Conversion of solid dicyclopentadiene to a liquid monomer for use in reaction injection molding**
Umsatz von festem Dicyclopentadien in einem flüssigen Monomer zur Verwendung beim Reaktionsspritzgiessen
Conversion de dicyclopentadiène solide en monomère liquide pour utilisation dans un moulage à réaction par injection

(30) Priority: 08.12.1986 US 939608; 26.02.1987 US 19465
(43) Date of publication of application: 15.06.1988
(73) Proprietor: National Starch and Chemical Investment Holding Corporation, Wilmington, Delaware 19803-7663 (US)
(72) Inventor: Espy, Herbert Hastings, Wilmington Delaware 19810 (US); Matlack, Albert Shelton, Hockessin Delaware 19707 (US)
(74) Representative: Hagemann, Heinrich, Dr.rer.nat., Dipl.-Chem.

(56) References cited:
- EP-A- 0 084 888
- EP-A- 0 181 640
- DE-A- 2 739 868
- US-A- 3 701 812
- US-A- 4 278 823
- US-A- 4 401 837
- US-A- 4 703 098
- B. Raistrick et al.:"Liquid-phase Reactions at High Pressures. Part V. The Polymerisation of Cyclopentadiene and alpha-Dicyclopentadiene." Journal of the Chemical Society, 1939, pp. 1761-1769

## Description

This invention relates to a method for preparing mixtures containing dicyclopentadiene that are liquid at room temperature, and to dicyclopentadiene copolymers.

U.S.-A-4,507,453 and 4,400,340 disclose thermoset polymers made by polymerization of dicyclopentadiene monomer, including copolymerization with other metathesis-polymerizable cyclic olefins. Those polymers and copolymers are noteworthy for their unusually good balance of high impact strength and high modulus. However, a drawback of poly(dicyclopentadiene) for many applications is that it has a relatively low heat distortion temperature and glass transition temperature (Tg), i.e., a Tg of about 120°C. It has been proposed to overcome this problem by copolymerizing dicyclopentadiene with comonomers such as dimethanooctahydronaphthalene, dimethanohexahydronaphthalene, and certain polar comonomers having multiple points of unsaturation. Such uncommon comonomers must be custom-synthesized and are much more expensive than dicyclopentadiene.

Thanks to their unusual balance of modulus and impact strength, dicyclopentadiene polymers and copolymers are commercially desirable materials, increasingly sought after for use in molding of shaped articles in complex configurations.

Such shaped articles are prepared at low temperatures and under low pressures using metathesis catalyst systems comprising a tungsten or molybdenum catalyst activated by an alkyl aluminum compound. They are formed by injecting the monomer, formulated with catalyst and activator components, into an appropriately shaped mold, wherein the monomer polymerizes. This can be done by a known two-stream process such as taught in the said U.S.-A-4,400,340 or, with appropriate moderation of the reaction rate, by a conventional one-stream process. However, those processes involve the problem that dicyclopentadiene melts at 32°C and is thus a solid at room temperature (about 25°C). Accordingly, it must be heated during its formulation with catalysts, activators and other additives, and transported through heat-jacketed lines to maintain a liquid state.

It is known that contaminants or adulterants of many kinds, when incorporated into a solid organic material, depress the melting point by a specific calculable amount. However, most adulterants that might be added to dicyclopentadiene in sufficient amounts to accomplish this reduction, monomeric cyclopentadiene for instance, interfere with or even prevent metathesis polymerization from taking place. Those that do not hinder polymerization generally have an undesirable effect on the properties of the polymer.

There is clearly a need for polymerizable dicyclopentadiene that is liquid at temperatures below about 25°C, preferably below 20°C, and particularly if it could be produced by an adulterant that is an oligomer (or mixture of oligomers) of cyclopentadiene that is copolymerizable with dicyclopentadiene, such as the trimer, tetramer or pentamer of cyclopentadiene. There is also a need for copolymers of dicyclopentadiene that have significantly better Tg and heat distortion temperatures than poly(dicyclopentadiene), but that do not require copolymers that are more expensive than dicyclopentadiene because they must be custom-made.

In the description that follows, the term "oligomers" or "cyclopentadiene oligomers" is intended to mean cyclopentadiene oligomers higher than dicyclopentadiene in molecular weight.

According to the invention, there is provided a polymerizable mixture containing dicyclopentadiene that is liquid at temperatures below normal handling temperatures (or the order of 20 to 25°C) and comprises about 40 to 95% by weight of dicyclopentadiene and about 60 to 5% by weight of higher cyclopentadiene oligomers, including the trimer of cyclopentadiene, is substantially free of cyclopentadiene and low molecular weight pyrolysis products derived from dicyclopentadiene, and can be polymerized under the influence of a metathesis catalyst system to form a crosslinked thermoset polymer.

If a low melting point is particularly important, the polymerizable mixture according to the invention preferably comprises not less than 8% by weight of from higher cyclopentadiene oligomers and not more than about 30%, most preferably not more than about 20%, of higher cyclopentadiene oligomers.

Also according to the invention, a method for preparing a metathesis-polymerizable liquid mixture containing dicyclopentadiene comprises subjecting dicyclopentadiene to a heat treatment at about 125°C to 250°C in the substantial absence or air under conditions that form cyclopentadient oligomers, preventing pyrolysis products from escaping from the reaction, and continuing the heating until a liquid mixture comprising about 40 to 95% by weight of dicyclopentadiene and about 60 to 5% by weight of higher cyclopentadiene oligomers is formed, and separating residual cyclopentadiene and low molecular weight pyrolysis products from the dicyclopentadiene and oligomer mixture to recover a product that can be polymerized under the influence of a methathesis catalyst system to form a crosslinked thermostat polymer.

Such polymerization normally and preferably is complete in about 30 seconds or less.

Again, if a low melting point is particularly important, in the said method for preparing a metathesis-polymerizable liquid mixture according to the invention the heating is continued until the liquid mixture comprises not less than 8% by weight of higher cyclopentadiene oligomers and not more than about 30%, most preferably not more than about 20%, of higher cyclopentadiene oligomers.

Preferably, according to the invention, the starting dicyclopentadiene has a high degree of purity. A substantially pure dicyclopentadiene, in a conventional two-stream metathesis polymerization as described in U.S. -A-4,400,340, polymerizes to a substantially cross-linked condition within one-half minute or less. Such dicyclopentadiene is 99⁺% dicyclopentadiene. A method of preparing it is described in U.S.-A-4,584,425. However, dicyclopentadiene of normally available purity level can be employed, since the mixture of dicyclopentadiene and oligomers can be purified as described in the said U.S.-A-4,584,425.

The thermal or pyrolytic conversion of dicyclopentadiene to higher oligomers is well known. The reaction proceeds by initial pyrolysis of dicyclopentadiene to cyclopentadiene followed by addition of cyclopentadiene to dicyclopentadiene to form tri-, tetra-, and higher cyclopentadiene oligomers. The reaction has traditionally been regarded as a nuisance, inasmuch as little use has conventionally been known for these oligomers, and the presence of residual cyclopentadiene and low molecular weight pyrolysis products would have prevented polymerization of the mixture even if such had been contemplated. But according to the invention, the presence of the oligomers is highly desirable for two reasons: their presence can be responsible for depressing the melting point of the dicyclopentadiene to a point at which the liquid state is maintained, and crosslinked copolymers of dicyclopentadiene with higher cyclopentadiene oligomers have glass transition and heat distortion temperatures significantly higher than does dicyclopentadiene homopolymer.

The best melting point depression is achieved within the range of about 8 to 20% oligomer content. Below about 5%, the mixture may be liquid at room temperature, but freezes at slightly lower temperatures, leaving only a small temperature range in which to operate. Above about 30%, the mixture begins to resemble an oligomer adulterated with dicyclopentadiene and melting point increases rapidly since the molar freezing point depression constant of dissolved dicyclopentadiene is much greater than that of dissolved oligomers. However, this disadvantage may be balanced against the improvement in copolymer characteristics that may result from somewhat higher proportions of cyclopentadiene oligomers. From that point of view, the copolymers according to this invention are preferably those having 40 to 85% dicyclopentadiene and 15 to 60% oligomer, and most preferred are those having 40 to 80% dicyclopentadiene and 20 to 60% oligomer.

Within the effective temperature range of about 125 to 250°C for conversion of dicyclopentadiene to oligomers according to this invention, the preferred temperature range for producing up to 30% by weight of oligomers is about 185 to 190°C and the most preferred is about 140 to 170°C. The time required to carry out the reaction can vary over a wide range depending on the oligomer content desired and also on the temperature range selected. Heating times required to yield some desirable oligomer contents are approximately as follows:

| Temperature | 5 - 30% Oligomer | 10 - 25% Oligomer |
|---|---|---|
| 140°C | 5-60 hrs. | 12-36 hrs. |
| 150°C | 2-24 hrs. | 5-15 hrs. |
| 170°C | 0.4-4 hrs. | 1-3 hrs. |
| 190°C | 0.1-1 hrs. | 0.15-0.75 hrs. |

Preferably, the process is carried out in the absence of any solvent. The reaction proceeds very well in the melt and recovery of the product is facilitated, since no solvent removal is required. However, the process can be carried out in the presence of a hydrocarbon solvent, either aliphatic or aromatic. The aromatic solvents are preferred.

The method of the invention, as stated, leads to the formation of tri-, tetra-, and higher cyclopentadiene oligomers, but for optimum predictability of the characteristics of the polymerization products, it is preferred that the proportion of the tricyclopentadiene relative to other oligomers be as great as possible.

Within the heating temperature range of 125 to 250°C, particularly within the preferred heating temperature range of 140 to 170°C, a time/temperature relationship (as shown in the preceding table) can be maintained such that the greater portion of the oligomer formed is tricyclopentadiene containing a small amount of tetracyclopentadiene and virtually no oligomers higher than tetracyclopentadiene. Higher temperatures and longer times lead to increased proportions of the oligomers being higher than tricyclopentadiene, but may be desirable for improving final polymer properties. Lower temperatures, say 125°C, lead to an even higher proportion of tricyclopentadiene, although at this low temperature, the heating time required is generally regarded as impractically long.

In order to make the reaction proceed, it is necessary that it be carried out under conditions such that the cyclopentadiene pyrolysis products, specifically cyclopentadiene, remain within the reaction mass. This is accomplished by operating under reflux conditions. When operating at temperatures about 140°C, a pressurized reflux system should be used.

Due to the presence of carbon-carbon unsaturation in dicyclopentadiene and also in the oligomers, the system is strongly susceptible to air oxidation. Thus the reaction is carried out in the absence of air and preferably in the presence of an antioxidant. Butylated hydroxytoluene (BHT) is a preferred antioxidant since it has been found to be compatible with the metathesis polymerization system. Other usable antioxidants will be known to the practitioner or can be determined by routine experimentation.

As in most organic chemical processes, there is always a small amount of low molecular weight by-products remaining after the pyrolysis reaction. These are usually low molecular weight, low boiling hydrocarbon materials, a high percentage of which can be unreacted cyclopentadiene monomer. Since these materials, particularly the cyclopentadiene, will interfere with the metathesis reaction and detract from the physical properties of the polymerization product that results, they must be removed.

Removal of by-products is accomplished by vacuum stripping at relatively low temperature, i.e., about 95 to 105°C and 6,65 KPa (50 mm HG) or less. Low temperature stripping is important so that pyrolysis of the previously formed oligomers is minimized. Inert gas sparging at about the same temperature can also be employed. Following the stripping operation, the dicyclopentadiene-oligomer mixture can be contacted with an absorbent such as alumina or a zeolite as described in U.S.-A-4,584,425.

The copolymers according to this invention are prepared via substantially the same techniques as have been taught for the preparation of dicyclopentadiene homopolymers, for instance using the technique described in U.S.-A-4,400,340. Thus, the reaction is carried out under the influence of a tungsten or molybdenum compound, activated by an alkyl aluminum compound. The preferred tungsten or molybdenum compounds are the halides. According to the process of the said patent, a plurality of reactive liquid streams are provided. One of these streams contains the metathesis catalyst component, preferably a tungsten halide and tungsten oxyhalide complex, dissolved in the dicyclopentadiene-oligomer mixture. Another stream contains the alkyl aluminum activator compound, preferably comprising an alkyl aluminum iodide, and a reaction rate moderator to delay the catalyst activation. The activator and moderator are also dissolved in the dicyclopentadiene-oligomer mixture.

The two streams are mixed and immediately injected into a mold of the desired shape where reaction takes place. The reaction rate moderator delays the onset of the reaction for the time required to transfer the entire mix into the mold, following which reaction is substantially completed within about half a minute or less.

Although the purified mixture of dicyclopentadiene and oligomer can be polymerized in about 30 seconds or less, by use of less active activators or more active reaction rate moderators, it is possible to delay the polymerization for substantial periods of time if that is desired. One technique for accomplishing such a delay is disclosed in EP-A-266587, which was filed earlier but published after the filing date of the present application; i.e. prior art according to Art. 54(3)(4)EPC.

It is, of course, not required that the dicyclopentadiene-oligomer mixture be prepared via the heat treatment in situ. If a source of isolated oligomers is available, the oligomers can be blended into the dicyclopentadiene at the desired ratios. The same type of melting point depression effect will be observed, and may be enhanced (depending on the composititon of the oligomers) and the same polymerization and molding techniques can be employed. This is the preferred way of operating when a content of oligomers greater than about 30% is desired. Room temperature handling may in this way be possible with a content of oligomers of 50% or even higher.

The significant property improvement in the final products of this invention is the improved glass transition temperature and heat distortion temperature of these copolymers compared to that of dicyclopentadiene homopolymer. The Tg of the homopolymer is on the order of about 120°C. The Tg of the copolymers increases as the oligomer content of the copolymers increases. As little as 5% of the oligomer in the product can increase the Tg by 10 or 12°C. At about 23% oligomer content, a Tg of greater than 160°C is observed. The same improvement is noted in the heat distortion temperature. Higher Tg and heat distortion temperature values greatly increase the scope of utility of the products.

It is believed that the increase of Tg and heat distortion temperature of these copolymers is caused by decreased mobility of the polymer claims due to the presence of the larger, more rigid oligomer molecules. For this same reason, however, the impact resistance of the polymer is significantly reduced compared to that of dicyclopentadiene homopolymer. As Tg is gained, impact resistance is correspondingly lost.

The lost impact resistance can be substantially regained in the final molded polymer product either by reinforcement or by including an elastomer in the formulation. About 3 to 15% by weight of an elastomer is already added to most dicyclopentadiene polymerizations for the purpose of increasing the viscosity of the reaction streams to facilitate nonturbulent filling of molds. The elastomer yields the additional beneficial effect of improving the impact resistance of the polymer product. Impact resistance of the rubbercontaining copolymer is sufficient for virtually all applications. Elastomers that can be employed include butyl rubber, polyisoprene, polybutadiene, polyisobutylene, styrene-butadiene block and random copolymer rubbers, ethylene-propylene copolymers and ethylene-propylene-diene terpolymers. Styrene-butadiene-random copolymer rubber is especially effective for increasing impact resistance. The amount of elastomer used is determined by its molecular weight and is limited by the maximum viscosity tolerable in the resultant reaction streams. That viscosity must not be so high as to interfere with rapid mixing of the reactant streams. The elastomer can be added to one or all of the reactant streams, but is preferably dissolved in all streams since mixing of the streams takes place most readily when all have essentially the same viscosity.

Reinforcement of the copolymer to increase its impact resistance can also be accomplished by addition of fibrous or particulate reinforcing materials. Particulate reinforcing materials include, inter alia glass, wollastonite, mica, carbon black, talc and calcium carbonate. Reinforcing materials can be added in amounts up to about 75% by weight based on the weight of the polymer, preferably about 1 to 40%. Another preferred form of reinforcement is organic or inorganic fibrous materials and, in particular, glass. The fiber can be either chopped, as staple fiber or in the form of a continuous mat. When the reinforcing material is fibrous, itis preferred to use a low viscosity reaction stream which can readily flow around and in among the fibers. For this reason, elastomer is frequently not included in the reactant streams in the fiber-filled embodiment.

The invention is illustrated in the following examples.

Aliquots (10 ml) of polymerization grade dicyclopentadiene (99⁺% pure) were injected into capped, N₂-sparged polymerization tubes. These tubes were placed snugly into holes in a cylindrical aluminum block, heated in a heating mantle. Temperature was controlled ^{±}3°C by a thermostatic control, monitoring a thermometer in the block. Reaction temperatures were 150°, 170° and 190°C. Zero time was counted when the block reached the specified reaction temperature. Tubes were removed at scheduled times thereafter and allowed to cool to room temperature.

Freezing behavior of the treated dicyclopentadiene was monitored by cooling the tubes in a) a cold room at 5°C, b) in ice, c) in a freezer at -20°C and d) in various dry ice solvent baths. Composition of the mixtures was determined by GC analysis with results recorded in Table A.

**Table A**

| Example No. | Temp. °C | Time Hr^{a} | Composition (GC Analysis) | | | | | Approx. M.P. °C |
|---|---|---|---|---|---|---|---|---|
| | | | Light Ends | DCPD | Co-Dimers | Cp Trimer | Cp Tetramer | |
| Control (Unheated) | | | 2.3 | 96.1 | 1.1 | 0.5 | 0.01 | |
| 1 | 150 | 0 | 2.1 | 96.7 | 0.9 | 0.04 | 0.02 | 20 |
| 2 | | 1 | 2.9 | 93.1 | 1.0 | 2.9 | 0.03 | 4 |
| 3 | | 2 | 3.6 | 90.8 | 1.0 | 4.4 | 0.03 | -20 |
| 4 | | 4 | 3.9 | 87.1 | 1.0 | 7.8 | 0.01 | -20 |
| 5 | | 8 | 3.7 | 80.8 | 0.9 | 13.5 | 0.9 | -22 |
| 6 | 170 | 0 | 2.9 | 95.2 | 1.1 | 0.16 | 0.03 | 4 |
| 7 | | 0.5 | 3.8 | 88.0 | 0.9 | 6.8 | 0.3 | -25 |
| 8 | | 1 | 4.2 | 85.2 | 0.9 | 8.8 | 0.6 | -22 |
| 9 | | 2 | 3.7 | 75.6 | 0.9 | 17.8 | 1.7 | -22 |
| 10 | 190 | 0 | 3.8 | 93.8 | 1.0 | 1.2 | 0.02 | -22 |
| 11 | | 0.25 | 3.3 | 76.8 | 0.8 | 16.8 | 2.0 | -22 |
| 12 | | 0.5 | 3.1 | 68.6 | 0.8 | 22.7 | 3.6 | -50 |

| | | | | | | | | |
|---|---|---|---|---|---|---|---|---|
| ^{a}Elapsed time after reaching indicated temperature. | | | | | | | | |

Several portions (1.5 liter) of the polymerization grade dicyclopentadiene were charged to a nitrogen-flushed agitated flask fitted with a reflux condenser and thermometer with thermostatic temperature controller. The reaction mixture was maintained under nitrogen while nitrogen gas was passed over the end of the reflux condenser with a bubbler. After four hours at 155°C or 6 hours at 150°C, the mass was cooled, passed through an alumina column under nitrogen and collected in nitrogen-flushed capped bottles containing about 300 ppm (based on the dicyclopentadiene-oligomer mixture) of butylated hydroxytoluene. Half of each preparation was heated to about 35°C and sparged with nitrogen to remove residual cyclopentadiene and other low boiling materials, then analyzed via Gas Chromatography.

The dicyclopentadiene/oligomer mixtures were polymerized into plaques about 30 cm square and 0.3 cm thick. Physical property measurements were made on these plaques.

The polymerization was carried out at follows:
A 0.5 M solution of tungsten catalyst was prepared by weighing 19.80 g (0.05 mole) of WCl₆ under nitrogen into a 200 ml bottle containing a teflon-coated magnetic stirring bar. The tungsten was then slurried in 90 ml of toluene that had been distilled from Na/K alloy under nitrogen. t-Butanol (0.925 g, 0.0125 moles) dissolved in 5 ml of toluene, was added, and the mixture was stirred for one hour while sparging with nitrogen. Nonyl phenol (11.05 g, 0.05 moles) dissolved in 5 ml of toluene was added, and the mixture was stirred for one hour while sparging with nitrogen. Acetyl-acetone (10.00 g, 0.100 moles) was then added by syringe and the mixture was stirred overnight while sparging with nitrogen to remove HCl gas. Toluene was then added to restore the volume of the solution to its original level and produce a 0.50 M solution.

A 1.0 M solution of complexed aluminum alkyl activator can be prepared by diluting 5.70 g of di-n-octylaluminum iodide, 31.17 g of tri-n-octylaluminum, and 13.42 g of bis(methoxyethyl)ether (diglyme) to a volume of 100 ml with distilled toluene, under a nitrogen atmosphere.

Into 300 ml of heat-treated dicyclopentadiene made by each of the four combinations: temperature - 150° and 155° - and sparging (done or not), is added about 4.5 ml of 1 molar aluminum alkyl activator complex and about 3.0 ml of 0.5 molar tungsten complex under a nitrogen atmosphere. After rapid mixing, the resulting solution of catalyst, and activator in dicyclopentadiene is quickly poured into the mold and allowed to polymerize. After the polymerization is complete and the plaque has cooled, the mold is dismantled and the plaque is removed.

Composition data for the dicyclopentadiene/oligomer mixture and properties of the resultant polymer are recorded in Table B.

**Table B**

| | Example Nos. | | | | Untreated Control |
|---|---|---|---|---|---|
| | 13 | 14 | 15 | 16 | |
| | | | | | |

| Preparation | | | | | |
|---|---|---|---|---|---|
| Heat Cycle Temp. °C | 155 | 150 | 150 | 155 | |
| Heat Cycle Time | 4 | 6 | 6 | 4 | |
| N₂ Sparged Afterwards | no | no | yes | yes | |

| Analyses | | | | | |
|---|---|---|---|---|---|
| Light Ends (incl. Cp* monomer), % | 0.29 | 0.33 | 0.23 | 0.08 | 0.06 |
| DCPD, % | 88.4 | 88.6 | 87.8 | 88.9 | 98.3 |
| Cp trimers, % | 9.9 | 10.2 | 11.0 | 10.4 | 1.6 |
| Cp tetramers, % | 0.5 | 0.4 | 0.6 | 0.5 | 0.01 |

| Freezing Tests (°C) | | | | | |
|---|---|---|---|---|---|
| All liquid | -9°C | -9°C | -5°C | -5°C | 30°C |
| Amt frozen at -8°C | | | 40% | 80% | 100% |
| Amt frozen at -16°C | 80% | 70% | | | |
| Amt frozen at -20°C | 100% | 100% | 100% | 100% | |

| Polymer Properties | | | | | |
|---|---|---|---|---|---|
| Tg, °C | 133 | 135 | 138 | 148 | 110-115 |
| Residual Monomer, % | 2.30 | 2.15 | 1.54 | 1.28 | |

| Mechanical Properties | | | | | |
|---|---|---|---|---|---|
| Flex Modulus, GPa | 2.29 | 2.30 | 2.27 | 2.22 | |
| Flex Strength, MPa | 90.9 | 90.9 | 87.5 | 88.5 | |
| Plate Impact, Joules | 4.05 | 3.77 | 4.16 | 1.30 | |
| Notched Izod, J/m | 215 | 199 | 211 | 69 | |

| | | | | | |
|---|---|---|---|---|---|
| *Cp = cyclopentadiene. | | | | | |

Polymerization grade dicyclopentadiene was heated in a steel autoclave under a nitrogen atmosphere at 145°C for 6 to 9 hours or 165°C for 3 to 5 hours. The heat-treated dicyclopentadiene was then vacuum stripped at about 6,66 kPa (50 mm Hg), at pot temperatures from about 98° to about 108°C, giving material with the analyses and freezing points shown in Table C.

Each of the four dicyclopentadiene-oligomer mixtures was polymerized using a standard reaction injection molding (RIM) machine (Accuratio Co., Jeffersonville, Indiana). The liquid dicyclopentadiene--oligomer mixture was blended with 6% by weight of styrene--butadiene random copolymer rubber (Stereon 720A) and charged to each of the monomer storage tanks on the RIM machine. The tanks were inerted with nitrogen. To one tank was added sufficient tri-n-octylaluminum to form a 0.0226 M solution, sufficient dioctylaluminum iodide to form a 0.004 M solution and sufficient dimethoxyethyl ether that the molar ratio of dimethoxyethyl ether to Al was 1:1. To the other tank was charged sufficient tungsten catalyst solution (prepared as in Example 1) to form a 0.0089 M solution in the monomer. Oxygen and moisture were excluded from the system throughout.

The two reactant solutions were mixed in equal proportions using a standard impingement type RIM mixing head through orifices of 0.081 cm at a flow rate of about 80 ml/second and a pressure of about 1000 psi. The mixture was pumped directly into a 25,4 cm x 25,4 cm x 0,3175 cm (10" x 10" x 1/8") plaque mold heated to between 50 and 60°C. Polymerization was complete and the plaque was demolded after about 30 seconds.

Physical properties of the resultant polymer are recorded in Table C.

**Table C**

| | Dicyclopentadiene Treatment and Composition | | | |
|---|---|---|---|---|
| Designation: XD-95 | 18 | 19 | 20 | 21 |
| Heating Temperature, °C | 145 | 145 | 165 | 165 |
| Heating Time, hr. | 6 | 9 | 3 | 5 |

| Composition After Stripping, % | | | | |
|---|---|---|---|---|
| Cp* Monomer | 0.02 | 0.06 | 0.03 | 0.06 |
| DCPD | 93.5 | 89.7 | 83.3 | 76.4 |
| Cp Trimer | 6.5 | 10.2 | 16.1 | 22.2 |
| Cp Tetramer | - | - | 0.6 | 1.3 |
| Freezing Point, °C | 6.4 | -30 | -38 | -30 |

| | Polymer Properties | | | |
|---|---|---|---|---|
| Tg, °C | 132 | 141 | 152 | 162 |
| Heat Distortion Temp., °C | 88 | 98 | 109 | 118 |
| % Swell | 100 | 90 | 84 | 80 |

| Mechanical Properties | | | | |
|---|---|---|---|---|
| Flex Modulus, GPa | 1.94 | 2.09 | 2.04 | 2.14 |
| Flex Strength, MPa | 78 | 85 | 87 | 90 |
| Tensile Modulus, GPa | 1.59 | 1.61 | 1.61 | 1.63 |
| Tensile Strength, MPa | 41 | 45 | 44 | 48 |
| Elongation, % | 79 | 60 | 39 | 50 |
| Plate Impact, Joules | 21.0 | 19.8 | 17.4 | 13.2 |
| Notched Izod, J/m | 384 | 281 | 198 | 133 |

| | | | | |
|---|---|---|---|---|
| *Cp = cyclopentadiene | | | | |

A quantity of dicyclopentadiene was heated at 165°C for 3 hours forming 16% cyclopentadiene oligomers. Residual dicyclopentadiene and low boiling pyrolysis products were stripped at approximately 100 to 110°C and 3,99 KPa (30 mm Hg). The residue had a melting point of 38°C and contained about 4% dicyclopentadiene, about 86.5% tricyclopentadiene, and about 9.5% tetracyclopentadiene.

Mixtures of this oligomer concentrate with dicyclopentadiene remained liquid at room temperature (25°C) at up to 50% concentrate by volume. Samples of dicyclopentadiene containing 10%, 25% or 50% of the above mixture containing 86.5% tricyclopentadiene and 9.5% tetracyclopentadiene all polymerized adequately when treated by the following procedure:
Five milliliters of monomer mixture (dicyclopentadiene--crude oligomer blend) was syringed into a test tube which had been capped and sparged with nitrogen. Aluminum alkyl activator solution (0.22 ml of a 0.4 M solution) was syringed into the test tube and mixed with the contents by shaking. Then 0.29 ml of a 0.1 M tungsten catalyst solution was added to the test tube by syringe, after which the contents were mixed by shaking vigorously. After about one minute a sharp exothermic polymerization was observed.

A quantity of dicyclopentadiene was heated at 165°C for about five hours. At that time residual dicyclopentadiene and low boiling pyrolysis products were stripped at 100 to 110°C and 3,99 KPa (30 mm Hg). A product containing approximately 42% dicyclopentadiene and 58% of an oligomer mixture consisting of approximately 51.5% tricyclopentadiene and 6.5% tetracyclopentadiene was recovered.

The mixture as recovered was polymerized by adding catalyst and activator to the monomer in an agitated bottle in a ratio of monomer/tungsten aluminum equal to 1,000/0.67/2.0. The mix was transferred to a glass plaque mold (described in Example 1) substantially immediately and allowed to polymerize. Polymerization was complete in about thirty seconds and the plaque was removed from the mold.

A portion of the mixture was diluted with dicyclopentadiene until the oligomer content was about 43%. This was polymerized in the same way.

Physical properties of these polymers are recorded in Table 3.

**Table D**

| | 58% | 43% |
|---|---|---|
| Flex Modulus, GPa | 2.64 | 2.54 |
| Flex strength, MPa | 110 | 103 |
| Heat distortion Temperature, °C | 145 | 139 |

## Claims

1. A mixture consisting of 40 to 95% by weight of dicyclopentadiene and 60 to 5% by weight of higher cyclopentadiene oligomers, which is liquid below 25°C.

2. A mixture as claimed in claim 1, further characterized in that it consists of 70 to 95% by weight of dicyclopentadiene and 30 to 5% by weight of higher cyclopentadiene oligomers, predominantly the trimer of cyclopentadiene.

3. A mixture as claimed in claim 2, further characterized in that it consists of 80 to 92% by weight of dicyclopentadiene and 20 to 8% by weight of the higher cyclopentadiene oligomers, predominantly the trimer of cyclopentadiene.

4. A mixture as claimed in claim 1, 2, or 3, further characterized in that its melting point is not over 20°C.

5. A mixture as claimed in claim 1, 2, 3, or 4, further characterized in that at least 85% of the higher cyclopentadiene oligomers is tricyclopentadiene.

6. A method for preparing a metathesis-polymerizable mixture as claimed in claim 1, 2, 3, 4, or 5, which comprises subjecting dicyclopentadiene to a heat treatment at 125°C to 250°C in the absence of air under reflux conditions wherein pyrolysis products are prevented from escaping from the reaction and cyclopentadiene oligomers are formed, continuing the heating until a liquid mixture comprising 40 to 95% by weight of dicyclopentadiene and 60 to 5% by weight of higher cyclopentadiene oligomers is formed and separating residual cyclopentadiene and low molecular weight pyrolysis products from the dicyclopentadiene and oligomer mixture to recover a product.

7. A method as claimed in claim 6, further characterized in that the heating is continued until a liquid mixture comprising 70 to 95% by weight of dicyclopentadiene and 30 to 5% by weight of higher cyclopentadiene oligomers is obtained.

8. A method as claimed in claim 7, further characterized in that the heating is continued until a liquid mixture comprising 80 to 92% by weight of dicyclopentadiene and 20 to 8% by weight of higher cyclopentadiene oligomers is obtained.

9. A method as claimed in claim 6, 7, or 8, further characterized in that the starting dicyclopentadiene is at least 99% dicyclopentadiene.

10. A method is claimed in claim 6, 7, 8, or 9, further characterized in that the heat treatment is carried out at 140°C to 170°C.

11. A method as claimed in claim 6, 7, 8, 9, or 10, further characterized in that the heat treatment is carried out in the absence of a solvent.

12. A method as claimed in claim 6, 7, 8, 9, 10, or 11, further characterized in that the heat treatment is carried out in the presence of an antioxidant.

13. A method as claimed in claim 12, further characterized in that the antioxidant is butylated hydroxytoluene (BHT).

14. Use of the liquid mixture containing dicyclopentadiene and higher cyclopentadiene oligomers as claimed in claim 1, 2, 3, 4, or 5 to produce by copolymerization a copolymer consisting of 40 to 95% by weight, based on total copolymer weight, of repeating units derived from dicyclopentadiene and 60 to 5% by weight of repeating units derived from higher cyclopentadiene oligomers.

## Patentansprüche

1. Mischung bestehend aus 40 bis 95 Gewichts-% Dicyclopentadien und 60 bis 5 Gewichts-% höhere Cyclopentadienoligomere, die unter 25°C flüssig ist.

2. Mischung nach Anspruch 1, weiterhin dadurch gekennzeichnet, daß sie aus 70 bis 95 Gewichts-% Dicyclopentadien und 30 bis 5 Gewichts-% höhere Cyclopentadienoligomere besteht, überwiegend das Trimer von Cyclopentadien.

3. Mischung nach Anspruch 2, weiterhin dadurch gekennzeichnet, daß sie aus 80 bis 92 Gewichts-% Dicyclopentadien und 20 bis 8 Gewichts-% höhere Cyclopentadienoligomere besteht, überwiegend das Trimer von Cyclopentadien.

4. Mischung nach Anspruch 1, 2 oder 3, weiterhin dadurch gekennzeichnet, daß ihr Schmelzpunkt nicht über 20°C liegt.

5. Mischung nach Anspruch 1, 2, 3 oder 4, weiterhin dadurch gekennzeichnet, daß mindestens 85 Gewichts-% der höheren Cyclopentadienoligomere Tricyclopentadien sind.

6. Verfahren zur Herstellung einer metathesepolymerisierbaren Mischung nach Anspruch 1, 2, 3, 4 oder 5, umfassend das Aussetzen des Dicyclopentadiens einer Wärmebehandlung bei 125°C bis 250°C in Abwesenheit von Luft unter Rückflußbedingungen, wobei verhindert wird, daß Pyrolyseprodukte aus der Reaktion austreten, und wobei Cyclopentadienoligomere gebildet werden, Fortsetzen der Erwärmung, bis eine flüssige Mischung umfassend 40 bis 95 Gewichts-% Dicyclopentadien und 60 bis 5 Gewichts-% höhere Cyclopentadienoligomere gebildet wird, und Abtrennen des restlichen Cyclopentadiens und der Pyrolyseprodukte mit niedrigem Molekulargewicht von der Dicyclopentadien- und Oligomermischung zur Gewinnung eines Produkts.

7. Verfahren nach Anspruch 6, weiterhin dadurch gekennzeichnet, daß das Erwärmen fortgesetzt wird, bis eine flüssige Mischung umfassend 70 bis 95 Gewichts-% Dicyclopentadien und 30 bis 5 Gewichts-% höhere Cyclopentadienoligomere erhalten wird.

8. Verfahren nach Anspruch 7, weiterhin dadurch gekennzeichnet, daß das Erwärmen fortgesetzt wird, bis eine flüssige Mischung umfassend 80 bis 92 Gewichts-% Dicyclopentadien und 20 bis 8 Gewichts-% höhere Cyclopentadienoligomere erhalten wird.

9. Verfahren nach Anspruch 6, 7 oder 8, weiterhin dadurch gekennzeichnet, daß das Ausgangsdicyclopentadien mindestens 99% Dicyclopentadien umfaßt.

10. Verfahren nach Anspruch 6, 7, 8 oder 9, weiterhin dadurch gekennzeichnet, daß die Wärmebehandlung bei 140°C bis 170°C durchgeführt wird.

11. Verfahren nach Anspruch 6, 7, 8, 9 oder 10, weiterhin dadurch gekennzeichnet, daß die Wärmebehandlung in Abwesenheit eines Lösungsmittels durchgeführt wird.

12. Verfahren nach Anspruch 6, 7, 8, 9, 10 oder 11, weiterhin dadurch gekennzeichnet, daß die Wärmebehandlung in Anwesenheit eines Antioxidationsmittels durchgeführt wird.

13. Verfahren nach Anspruch 12, weiterhin dadurch gekennzeichnet, daß das Antioxidationsmittel butyliertes Hydroxytoluol (BHT) ist.

14. Verwendung der flüssigen Mischung enthaltend Dicyclopentadien und höhere Cyclopentadienoligomere nach Anspruch 1, 2, 3, 4 oder 5 zur Herstellung eines Copolymers durch Copolymerisation bestehend aus 40 bis 95 Gewichts-%, basierend auf dem Gesamtgewicht des Copolymers, von sich wiederholenden Einheiten, die von Dicyclopentadien abgeleitet sind, und 60 bis 5 Gewichts-% von sich wiederholenden Einheiten, die von höheren Cyclopentadienoligomeren abgeleitet sind.

## Revendications

1. Mélange consistant en 40 à 95% en poids de dicyclopentadiène et 60 à 5% en poids d'oligomères supérieurs de cyclopentadiène, qui est liquide en dessous de 25°C.

2. Mélange selon la revendication 1, caractérisé en ce qu'il consiste en 70 à 95% en poids de dicyclopentadiène et 30 à 5% en poids d'oligomères supérieurs de cyclopentadiène, et de façon prédominante, du trimère de cyclopentadiène.

3. Mélange selon la revendication 2, caractérisé en ce qu'il consiste en 80 à 92% en poids de dicyclopentadiène et 20 à 8% en poids des oligomères supérieurs de cyclopentadiène, et de façon prédominante, du trimère de cyclopentadiène.

4. Mélange selon l'une quelconque des revendications 1 à 3, caractérisé en ce que son point de fusion n'est pas supérieur à 20°C.

5. Mélange selon l'une quelconque des revendications 1 à 4, caractérisé en ce qu'au moins 85% des oligomères supérieurs de cyclopentadiène consistent en tricyclopentadiène.

6. Procédé de préparation d'un mélange polymérisable par métathèse selon l'une quelconque des revendications 1 à 5, qui comprend le traitement thermique de dicyclopentadiène à une température comprise entre 125 et 250°C en l'absence d'air, dans des conditions de reflux, dans lequel on empêche les produits de pyrolyse de s'échapper du milieu réactionnel, et les oligomères de cyclopentadiène sont formés, la poursuite du chauffage jusqu'à ce qu'un mélange liquide comprenant 40 à 95% en poids de dicyclopentadiène et 60 à 50% en poids d'oligomères supérieurs de cyclopentadiène soit formé, et la séparation du cyclopentadiène résiduel et des produits de pyrolyse de faible poids moléculaire du mélange de dicyclopentadiène et d'oligomère pour récupérer un produit.

7. Procédé selon la revendication 6, caractérisé en ce que le chauffage est poursuivi jusqu'à ce qu'un mélange liquide comprenant 70 à 95% en poids de dicyclopentadiène et 30 à 5% en poids d'oligomères supérieurs de cyclopentadiène soit obtenu.

8. Procédé selon la revendication 7, caractérisé en ce que le chauffage est poursuivi jusqu'à ce qu'un mélange liquide comprenant 80 à 92% en poids de dicyclopentadiène et 20 à 8% en poids d'oligomères supérieurs de cyclopentadiène soit obtenu.

9. Procédé selon l'une quelconque des revendications 6 à 8, caractérisé en ce que le dicyclopentadiène de départ consiste en dicyclopentadiène à raison d'au moins 99%.

10. Procédé selon l'une quelconque des revendications 6 à 9, caractérisé en ce que le traitement thermique est effectué à une température comprise entre 140 et 170°C.

11. Procédé selon l'une quelconque des revendications 6 à 10, caractérisé en ce que le traitement thermique est effectué en l'absence de solvant.

12. Procédé selon l'une quelconque des revendications 6 à 11, caractérisé en ce que le traitement thermique est effectué en présence d'un antioxydant.

13. Procédé selon la revendication 12, caractérisé en ce que l'antioxydant est l'hydroxytoluène butylé (BHT).

14. Utilisation du mélange liquide contenant du dicyclopentadiène et des oligomères supérieurs de cyclopentadiène selon l'une quelconque des revendications 1 à 5, pour préparer par copolymérisation un copolymère consistant en 40 à 95% en poids, par rapport au poids total de copolymère, de motifs répétitifs dérivés de dicyclopentadiène, et 60 à 5% en poids de motifs répétitifs dérivés d'oligomères supérieurs de cyclopentadiène.
